(19) Europäisches Patentamt
European Patent Office
Office européen des brevets

(11) EP 0 619 300 B1

(12) **EUROPÄISCHE PATENTSCHRIFT**

(45) Veröffentlichungstag und Bekanntmachung des Hinweises auf die Patenterteilung:
**27.12.1996 Patentblatt 1996/52**

(51) Int Cl.6: **C07C 255/40**, C07C 253/14, C07C 255/32

(21) Anmeldenummer: **94104664.1**

(22) Anmeldetag: **24.03.1994**

(54) **Verfahren zur Herstellung von Halogenmethylbenzoylcyaniden und neue Halogenmethyl-benzoylcyanide**

Process for the preparation of halogenmethylbenzoyl cyanides and halogenmethyl benzoylcyanides

Procédé de préparation de halogèneméthyl benzoylcyanides et halogèneméthyl benzoylcyanides

(84) Benannte Vertragsstaaten:
**AT BE CH DE DK FR GB IT LI NL**

(30) Priorität: **08.04.1993 DE 4311722**

(43) Veröffentlichungstag der Anmeldung:
**12.10.1994 Patentblatt 1994/41**

(73) Patentinhaber: **BASF Aktiengesellschaft**
**67063 Ludwigshafen (DE)**

(72) Erfinder:
- **Isak, Heinz Dr.**
  **D-67459 Boehl-Iggelheim (DE)**
- **Wettling, Thomas Dr.**
  **D-67117 Limburgerhof (DE)**
- **Keil, Michael Dr.**
  **D-67251 Freinsheim (DE)**
- **Wolf, Bernd Dr.**
  **D-67136 Fussgoenheim (DE)**
- **Doetzer, Reinhard Dr.**
  **D-69469 Weinheim (DE)**

(56) Entgegenhaltungen:
**EP-A- 0 142 797**
**EP-A- 0 398 783**
**GB-A- 1 524 660**
**US-A- 4 238 413**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 619 300 B1

Printed by Jouve, 75001 PARIS (FR)

**Beschreibung**

Die vorliegende Erfindung betrifft ein Verfahren zur Herstellung von Halogenmethyl-benzoylcyaniden der allgemeinen Formel I

PH-CO-CN    I

wobei PH einen Phenylrest bedeutet, der durch Chlormethyl oder Brommethyl substituiert ist und der gewünschtenfalls noch 1 bis 4 weitere, für die Umsetzung inerte Reste tragen kann.

Es ist allgemein bekannt, daß Benzoylcyanid u.a. durch Umsetzung von Benzoylchlorid

- mit Quecksilbercyanid (F. Wöhler und J. Liebig, Annalen der Chemie <u>3</u>, 249, 267 (1832)),
- mit Silbercyanid (Liebigs' Annalen der Chemie <u>287</u>, 307 (1895)),
- mit trockenem Kupfer(I)cyanid (Org. Synthesis <u>24</u>, 14 (1944)) oder
- mit trockenem Cyanwasserstoff in Gegenwart von Pyridin (Chem. Ber. <u>31</u>, 1023 (1898)) hergestellt werden kann.

In der EP-A 352 543 werden substituierte Benzoylcyanide (siehe Formel II auf Seite 4 der Beschreibung und Anspruch 5) u.a. als mögliche Ausgangprodukte für herbizide 4-Phenylpyrazole genannt (siehe Formelschema (4) auf Seite 10 der Beschreibung). Eine Methode zur Herstellung dieser Benzoylcyanide wird jedoch nicht angegeben. Außer einer allgemeinen Formel für die Benzoylcyanide ist auch keine Einzelverbindung geoffenbart.

Aus der DE-A 40 42 282 ist die Herstellung von 2-Phenoxymethylbenzoylcyaniden durch Umsetzung von 2-Phenoxymethyl-benzoyl-chloriden mit Alkalimetall- oder Erdalkalimetallcyaniden, gewünschtenfalls in Anwesenheit von Blausäure, bekannt. Dieses Verfahren scheint jedoch ungeeignet für die Herstellung der Halogenmethyl-benzoylcyanide I zu sein, da entsprechend der Lehre von Houben-Weyl, Methoden der Organischen Chemie, Band VIII, 4. Auflage, Georg Thieme Verlag, Stuttgart 1952, S. 294, eine α-Halogenalkyl-Seitenkette an einem Aromaten sehr leicht mit Alkalimetallcyaniden reagiert. Beispielhaft wird hierzu die Umsetzung von Benzylchlorid mit Natriumcyanid angeführt. Außerdem wird auf die aus J. Am. Chem. Soc. <u>68</u>, 2741 (1946) bekannte Umsetzung von Benzylchlorid mit Kupfer(I) cyanid bei 150°C unter Ausschluß von Wasser hingewiesen.

In Analogie zu der aus Houben-Weyl bekannten Methode wäre demnach bei der Umsetzung der Halogenmethyl-benzoylchloride II mit Alkalimetall- oder Übergangsmetallcyaniden ein Produktgemisch aus Cyanomethyl-benzoylchlorid und Halogenmethyl-benzoylcyanid zu erwarten.

Aufgabe der vorliegenden Erfindung war es demnach, ein technisch brauchbares Verfahren zur Herstellung der Verbindung I bereitzustellen.

Demgemäß wurde das vorliegende Verfahren zur Herstellung von Halogenmethyl-benzoylcyaniden gefunden, welches dadurch gekennzeichnet ist, daß man ein Halogenmethyl-benzoylchlorid der Formel II

PH-CO-Cl    II

mit einem Alkalimetallcyanid oder Übergangsmetallcyanid umsetzt.

Die Halogenmethyl-benzoylchloride II lassen sich nach bekannten Halogenierungs-Methoden aus den entsprechenden Methyl-substituierten Benzol-Derivaten (vgl. z.B. DE-A 28 35 440 ) oder aus den entsprechenden Benzoesauren (vgl. z.B. DE-A 40 42 282) herstellen:

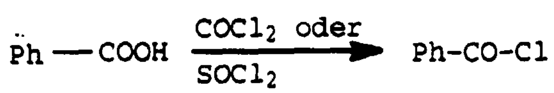


Das erfindungegemaße Verfahren wird normalerweise bei Atmospharendruck oder bei leichtem Unterdruck vorgenommen, wobei sich eine Reaktionstemperatur von (-20) bis 100°C, vorzugsweise 0 bis 80°C, insbesondere 20 bis 80°C, empfiehlt.

Unter den Alkalimetallcyaniden sind Natrium- und Kaliumcyanid bevorzugt. Unter den Übergangsmetallcyaniden

eignen sich beispielsweise Quecksilber (I) cyanid, Silbercyanid und vorzugsweise Kupfer (I) cyanid.

Im allgemeinen werden das Halogenmethyl-benzoylchlorid II und das Alkalimetall- bzw. Übergangsmetallcyanid in etwa stöchiometrischen Mengen eingesetzt. Bevorzugt ist jedoch ein Überschuß an Cyanid bis zur 2-fachen Menge, insbesondere an 1,05- bis 1,5-fächer Menge, bezogen auf die Menge an II.

Sofern das eingesetzte Halogenmethyl-benzoylchlorid II nicht in flüssiger Form vorliegt, ist der Zusatz eines inerten organischen Lösungs- oder Verdünnungsmittels empfehlenswert, wobei insbesondere aprotische dipolare und unpolare Lösungsmittel geeignet sind.

Unter aprotisch-dipolaren Lösungsmitteln sind solche Lösungs-mittel zu verstehen, bei denen ein Lösungsmittelmolekül ein ausgeprägtes Dipolmoment besitzt, jedoch keine Wasserstoffatome trägt, welche zur Ausbildung von Wasserstoffbrücken befähigt sind. Die Dielektrizitätskonstante solcher Lösungsmittel ist größer als 15. Bezüglich der Definition für aprotisch-dipolare Lösungsmittel sei auf A. J. Parker, Chem. Rev. 69 (1969), Seiten 1 - 32, insbesondere Seite 2, verwiesen.

Geeignete aprotisch-dipolare Lösungsmittel sind beispielsweise Sulfoxide wie Dimethylsulfoxid, Diäthylsulfoxid, Dimethylsulfon, Diäthylsulfon, Methyläthylsulfon und Tetramethylensulfon; Nitrile wie Acetonitril, Benzonitril, Butyronitril, Isobutyronitril und m-Chlorbenzonitril; N,N-Dialkyl-substituierte Carbonamide wie Dimethylformamid, Tetramethylharnstoff, N,N-Dimethylbenzamid, N,N-Dimethylacetamid, N,N-Dimethylphenylacetamid, N,N-Dimethylcyclohexancarbonsäureamid, N,N-Dimethylpropionsäureamid und homologes Carbonsäurepiperidid, Carbonsäuremorpholid sowie Carbonsaurepyrrolidid, die entsprechenden N,N-Diathyl-, N,N-Dipropyl-, N,N-Diisopropyl-, N,N-Diisobutyl-, N,N-Dibenzyl-, N,N-Diphenyl-, N-Methyl-N-phenyl-, N-Cyclohexyl-N-methyl-, N-Äthyl-N-tert.-butyl-Verbindungen der vorstehend genannten N,N-Dimethylverbindungen, des weiteren N-Methyl-formanilid, N-Äthylpyrrolidon, N-Butylpyrrolidon, N-Äthyl-piperidon-(t), N-Methylpyrrolidon und Hexamethylphosphorsäuretriamid. Auch Gemische der genannten Solventien kommen in Betracht.

Bevorzugt sind Dimethylacetamid, N-Methylpyrrolidon, Dimethylformamid, Dimethylsulfoxid Tetramethylensulfon, Aceton und Acetonitril.

Geeignete unpolare Lösungsmittel sind vorzugsweise aromatische Kohlenwasserstoffe wie Benzol, Toluol und o-, m-, p-Xylol, chlorierte Kohlenwasserstoffe wie Methylenchlorid und Alkohole wie Methanol und Ethanol. Besonders bevorzugt ist Toluol.

Der Verfahrensverlauf kann durch Zugabe eines Katalysators günstig beeinflußt werden. Normalerweise ist die 0,005- bis 2-fache Menge, insbesondere die 0,01- bis 0,5-fache Menge, an Katalysator, bezogen auf die Menge an II, ausreichend.

Als Katalysatoren eignen sich hierfür allgemein die Halogenide, Cyanide, Hydroxide, Hydrogensulfate, $C_1$-$C_4$-Alkylsulfate und Tetrafluoroborate von quartären Stickstoff-Verbindungen sowie Aryl- und Alkyl-Phosphoniumhalogenide, also beispielsweise

- Tetra-($C_1$-$C_4$-alkyl)ammoniumhalogenide wie Tetraethylammoniumchlorid, Tetraethylammoniumbromid, Tetrapropylammoniumbromid, Tetrabutylammoniumchlorid, Tetrabutylammoniumbromid, Tetrabutylammoniumiodid, Tetraoctylammoniumbromid, Tributylmethylammoniumchlorid, Hexadecyltrimethylammoniumbromid, Hexadecyltrimethylammoniumchlorid-Lösung, Ethylhexadecyldimethylammoniumbromid, Methyltrioctylammoniumchlorid,

- die Salze der folgenden Ammonium-, Piperidinium- und Morpholiniumionen mit Ethylsulfat oder Bromid als Gegenion: Cyclohexyl-diethyl-n-butylammonium, $C_1$-$C_6$-Alkylbenzyldimethylammoniumchloride, Benzyltributylammoniumbromid, Benzyltributylammoniumchlorid, Benzyltriethylammoniumchlorid, Benzyltrimethylammoniumchlorid, Benzyl-cyclohexyl-diethylammonium, Benzyl-di-n-propyl-ethylammonium, Benzyl-di-n-butyl-ethyl ammonium, Benzyl-butyl-cyclohexyl-ethylammonium, Butyl-di(methoxyethyl)-ethylanmonium, Benzyl-dimethoxyethyl-ethylammonium, Dibenzyl-di-n-propylammonium, Dibenzyl-di-n-butylammonium, Di-n-butyl-di(methoxyethyl)ammonium, Benzyl-n-butyl-di(methoxyethyl)ammonium, Dibenzyl-di(methoxyethyl)ammonium, N-(n-butyl)-N-ethyl-piperidinium, N-Benzyl-N-ethyl-piperidinium, 4-(n-butyl)-4-ethyl-morpholinium, 4-Benzyl-4-ethyl-morpholinium, N-Di (n-butyl)piperidinium, N-Benzyl-N-(n-butyl)-piperidinium, 4-Di(n-butyl)-morpholinium, 4-Benzyl-4-(n-butyl)-morpholinium, Cyclohexyl-dibenzyl-ethylammonium, N,N-Di(n-butyl)-hexamethyleniminium, N-Benzyl-N-(n-butyl)-hexamethyleniminium, N,N-Dibenzyl-hexamethyleniminium, N,N-Dibenzylpiperidinium, 4,4-Dimethylmorpholinium, N,N-Di(n-butyl)pyrrolidinium, N-Benzyl-N-(n-butyl)-Pyrrolidinium, N,N-Dibenzylpyrrolidinium, N-Benzyl-N-hexylpiperidinium, N-Benzyl-N-(2-methylpentyl)-piperidinium, N-Benzyl-N-(2-ethylhexyl)-piperidinium, 1,1-Di(n-Butyl)-2-ethylpiperidin, l-Benzyl-l-(n-butyl)-2-ethylpiperidin, 4-Benzyl-4-hexyl-morpholinium, 4-(n-Butyl)-4-hexyl-morpholinium, 5 4-Benzyl-4-(2-ethylhexyl)morpholinium, N-(n-Butyl)-N-isobutyl-hexamethylenimin, N-Benzyl-N-isobutylhexamethylenimin, N-Isoanyl-N-benzyl-hexamethylenimin, N-Benzyl-N-(n-butyl)-3,3,5-trimethylhexamethylenimin, N-Benzyl-N-(2-methylbutyl)-piperidinium, N-Benzyl-N-(3-methylbutyl)-piperidinium, N-Benzyl-N-(2-methyl-3-methylbutyl)-piperidinium, N-Cyclopentylpiperidinium, N-Benzyl-N-(2-methyl-3-ethoxypropyl)-piperidinium, Cyclohexyl-diethyl- (n-butyl)-ammonium, 4-Benzyl-4-(2-methylamyl)-morpholinyl, 1-Benzyl-1-(n-butyl)-2-(n-butyl)-2-ethyl-piperidinium, N-Benzyl-N-ethyl-hexamethyleniminium, N-Benzyl-N-(2-methylamyl)-hexamethyleniminium, N-Benzyl-N-(3,5-dimethyl-5-methylhexyl)-hexamethyleniminium, N-Benzyl-N-(2-methylamyl)-3,3,5-trimethylhexamethyleniminium, N-Benzyl-N-(2-ethylhexyl)-3,3,5-trimethylhexamethyleniminium, N-Benzyl-N-cyclohexylhexamethyleniminium, N-Benzyl-N-(2-methyl-2-methoxyethyl)-hexamethyleniminium, N-Benzyl-N-(2-methyl-2-n-butoxyethyl)-hexamethyleniminium, N-Benzyl-N-[2-methyl-2-(2-methoxyethoxy)ethyl]-hexamethyleniminium, N-Benzyl-N-(2,5-dimethyl-2-propoxyethyl)-hexamethyleniminium, N-Benzyl-N-(2-methyl-3-methylbutyl)-3,3,5-trimethylhexamethyleniminium, N-Benzyl-N-(tetrahydropyran-2-yl-methyl) -piperidinium, N-Benzyl-N- (2-methyl-2-methoxyethyl)-piperidinium, N-Benzyl-N-(2-methyl-2-n-butoxyethyl)-piperidinium, N-Benzyl-N-[2-methyl-2-(2-methoxyethoxy)ethyl]-piperidinium, N-Benzyl-N-(4,4-dimethylamyl)-piperidinium, N-Benzyl-N-(tetrahydropyran-2-ylmethyl)-hexamethyleniminium, N-Benzyl-N-(2-methyl-3-methylbutyl)-hexamethyleniminium, N-Benzyl-N-(2-methyl-3-methylbutyl)-pyrrolidinium, 4-Benzyl-4-(2-methyl-3-methylbutyl)-morpholinium, NBenzyl-N-(n-propyl)-hexamethyleniminium, N-Benzyl-N-(isopropyl)-hexamethyleniminium, n-Butyl-(2-methylbutyl)-di(2-methoxyethyl)ammonium, Benzyl-(2-methylbutyl)-di(2-methoxyethyl)ammonium, (Bis-Ethylbutylbenzylammonium)-hexan, n-Butyl-(3-methylbutyl)-di(2-methoxyethyl)ammonium, N-Benzyl-(3-methylbutyl)-di(2-methoxyethyl)ammonium, 1,ω-Di-(N-ethyl-hexamethyleniminium)hexyl, 1,ω-Di-(N-benzyl-hexamethyleniminium)hexyl, 1,ω-Di-(N-n-butyl-hexamethyleniminium)hexyl, 1,ω-Di-(N-ethylhexamethyleniminium)octyl, 1,ω-Di-(N-benzyl-hexamethyleniminium)octyl, 1,ω-Di-(N-n-butyl-hexamethyleniminium)octyl, 1,ω-Di-(N-ethylpiperidinium)hexyl, 1,ω-Di-(N-benzyl-piperidinium)hexyl, 1,ω-Di-(N-n-butyl-piperidinium)hexyl, 1,ω-Di-(N-ethylpiperidinium)octyl, 1,ω-Di-(N-benzyl-piperidinium)octyl, 1,ω-Di-(N-n-butyl-piperidinium)octyl, 1,ω-Di-(N-ethylpyrrolidinium)hexyl, 1,ω-Di-(N-benzyl-pyrrolidinium)hexyl, 1,ω-Di-(N-n-butyl-pyrrolidinium)hexyl, 1,ω-Di-(N-ethylpyrrolidinium)octyl, 1,ω-Di-(N-benzyl-pyrrolidinium)octyl, 1,ω-Di-(N-n-butyl-pyrrolidinium)octyl, Dibutylethyl-phenethylammonium, n-Butyl-2-methylbutyl-di-(2-methoxyethyl)ammonium, Di-(2-methoxyethyl)-di-(n-propyl)ammonium, Di-(2-methoxyethyl)-diamylammonium, (-)-N-Benzylquininiumchlorid und (-)-N-Dodecyl-N-methylephedriniumbromid,

- Tetra($C_1$-$C_4$-alkyl) ammoniumcyanide wie Tetraethylammoniumcyanid und Tetrabutylammoniumcyanid, sowie Tetraethylammoniumhydroxid-Lösung, Tetrabutylammoniumhydroxid-Lösung, Benzyltriethylammoniumhydroxid-Lösung, BenzyltrimethylammoniumhydroxidLösung, Benzyltrimethylammoniumhydroxid-Lösung, Tetrabutylammoniumfluoridtrihydrat, Tetrabutylammoniumhydrogensulfat, Tetrabutylammoniumtetrafluoroborat, Tetraethylammoniumfluoroborat,

- Alkyl- und Aryl-substituierte Phosphoniumhalogenide wie Ethyltrioctylphosphoniumbromid, Tributylhexadecylphosphoniumbromid, Tetrabutylphosphoniumbromid, Tetrabutylphosphoniumchlorid-Lösung, Butyltriphenylphosphoniumchlorid, Tetraphenylphosphoniumbromid und Tetraphenylphosphoniumchlorid.

Nach den bisherigen Erkenntnissen sind Tetrabutylammoniumchlorid, Tetrabutylammoniumbromid und die Tetraalkylammoniumcyanide besonders gut geeignet. Sie werden vorzugsweise als etwa 50 gew.-%ige wässrige Lösungen eingesetzt.

Liegt das Alkalimetallcyanid in wässriger Lösung und das Halogenmethyl-benzoylchlorid II in einer organischen Phase vor, so empfiehlt es sich, die Reaktion durch Zugabe eines Phasentransfer-Katalysators zu beschleunigen. Als Phasentransfer-Katalysatoren sind hierfür Kronenether wie Benzo-[15]krone-5, Tris-[2-(2-methoxyethoxy)ethyl]-amin, Dicyclohexyl-[18]krone-6 und [18]Krone-6[18]Krone-6-tetracarbonsäure besonders gut geeignet.

Der Phasentransfer-Katalysator wird zweckmäßigerweise in einer Menge von 0,001 bis 1-mol-%, insbesondere von 0,001 bis 0,08 mol-%, bezogen auf die Menge an II, eingesetzt.

Das erfindungsgemäße Verfahren kann sowohl diskontinuierlich als auch kontinuierlich durchgeführt werden. Bei der kontinuierlichen Arbeitsweise leitet man die Reaktionspartner beispielsweise durch einen Rohrreaktor oder über Rührkesselkaskaden.

Die Verfahensprodukte I können auf übliche Weise, z.B. mittels Destillation, gereinigt werden.

Nach dem erfindungsgemäßen Verfahren erhält man die Halogenmethyl-benzoylcyanide I auf technisch einfache Weise mit sehr guter Reinheit. Die bei der Herstellung der 2-Halogenmethyl-benzoylcyanide als Nebenprodukt anfallende 2-Halogenmethyl-Benzoesäure kann in Phthalid überführt werden, das wieder zur Herstellung von II verwendet werden kann.

Im Hinblick auf die gewünschten Verfahrensprodukte I bedeutet Ph vorzugsweise einen Rest

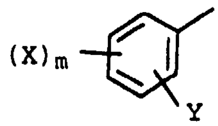

In den Verfahrensprodukten I und den neuen Halogenmethyl-benzoylcyaniden der Formel I' stehen

X für Halogen, insbesondere Fluor und Chlor, $C_1$-$C_4$-Alkyl, insbesondere Methyl und Ethyl, $C_1$-$C_4$-Alkoxy, insbesondere Methoxy, Ethoxy und Isopropoxy, $C_1$-$C_4$-Halogenalkyl, insbesondere Trifluormethyl, -C($C_1$-$C_5$-Alkyl)=N-O-($C_1$-$C_5$-alkyl) oder -C($C_1$-$C_5$-Alkyl)=N-O-($C_2$-$C_5$-alkenyl), insbesondere Methylhydroxylimino und -C($CH_3$)=N-$OCH_3$;

m für 0 bis 4, vorzugsweise 0 und

Y für Chlormethyl oder Brommethyl, vorzugsweise in 2-Position, wobei Chlormethyl besonders bevorzugt ist.

Die Halogenmethyl-benzoylcyanide I und I' sind wertvolle Zwischenprodukte zur Herstellung verschiedener Pflanzenschutzmittel, beispielsweise den herbiziden 4-Phenylpyrazolen, wie sie in der EP-A 352 543 beschrieben werden.

Die Verfahrensprodukte I und I' können außerdem zur Synthese von Arylglyoxylsäureestern gemäß der DE-A 40 42 271 dienen. Das aus der dort beschriebenen Pinner-Reaktion erhaltene Rohprodukt-Gemisch aus Phenylglyoxylsaureestern und deren Ketalen kann gemäß der Lehre der DE-A 40 42 272 ohne weitere Reinigung in die E-Oximether von Phenylglyoxylsäureestern der Formel

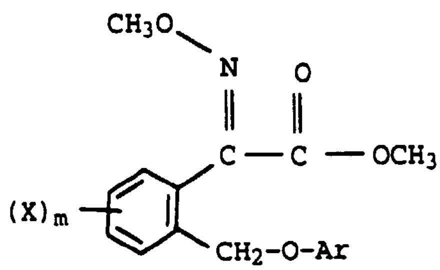

in der Ar für substituiertes oder unsubstituiertes Phenyl steht, ubergeführt werden. Verbindungen der Formel III finden im Pflanzenschutz vorzugsweise als Fungizide, Akarizide oder Insektizide Verwendung (vgl. z.B. EP-A 253 213 und EP-A 254 426).

Herstellungsbeispiele

Beispiel 1:

Herstellung von 2-Chlormethyl-benzoylcyanid unter Phasentransferbedingungen in Wasser/Toluol

Zu einer Lösung von 117,6 g (2,4 mol) Natriumcyanid in 400 g wasser wurde 1 g (3 mmol) Tetrabutylammoniumbromid gegeben. Dann stellte man die erhaltene Mischung mit ca. 200 g 1 gew.-%iger Salzsäure auf einen pH-Wert von 10,5 ein, wonach 1000 g Toluol zugegeben wurden. In dieses 2-Phasengemisch wurde innerhalb von 30 min eine Lösung von 378 g (2,0 mol) 2-Chlormethyl-benzoylchlorid in 1000 ml Toluol dosiert.

Nach ca. 2-3 Std. Rühren bei 25-35°C trennte man die Phasen. Die organische Phase wurde je einmal mit 200 ml Wasser und 200 ml 1 gew.-%iger Salzsäure gewaschen, danach über Magnesiumsulfat getrocknet und eingeengt. Das Rohprodukt wurde mittels fraktionierter Destillation (Sdp.: 100°C, 0,3mbar) gereinigt. Ausbeute: 292,34 g (82 %)

Beispiel 2:

Herstellung von 2-Chlormethyl-benzoylcyanid unter in Acetonitril/ Wasser

Eine Lösung von 9,45 g (0,05 mol) 2-Chlormethyl-benzoylchlorid in 100 ml wasserfeuchtem Acetonitril wurde mit 4,9 g (0,1 mol) Natriumcyanid versetzt, wonach man das Reationsgemisch 24 Std. bei etwa 25-30°C rührte. Die HPLC-Auswertung ergab 19 % einer Mischung aus Phthalid und 2-Chlormethyl-benzoesäure, 76 % 2-Chlormethylbenzoylcyanid und 5 % dimeres Benzoylcyanid.
Das Rohproduktgemisch konnte mittels Chromatographie an Kieselgel oder mittels fraktionierter Destillation (s. Beispiel 1) aufgetrennt werden.
Ausbeute: 6,3 g (70,5 %), $Sdp_{0,3}$ = 100°C

Beispiel 3:

Herstellung von 2-Chlormethyl-benzoylcyanid mit Cu-I-CN

Eine Losung von 9,45 g (0,05 mol) 2-Chlormethyl-benzoylchlorid in 100 ml Acetonitril wurde mit 8,9 g (0,1 mol) Cu(I)cyanid versetzt, wonach das Reaktionsgemisch 24 Std. bei ca. 0°C gerührt wurde. Die HPLC-Auswertung ergab 24 % Phthalid, 62 % 2-Chlormethyl-benzoylcyanid und ca. 10 % dimeres Benzoylcyanid.

Das Reaktionsgemisch konnte durch eine Chromatographie an Kieselgel (Laufmittel: Hexan/Toluol = 1:1) oder durch eine fraktionierte Destillation gemäß Beispiel 1 ($Sdp_{0,5}$ = 106°C) getrennt werden.
Ausbeute: 5,27 g (58 %)

**Patentansprüche**

1. Verfahren zur Herstellung von Halogenmethyl-benzoylcyaniden der allgemeinen Formel I

$$\text{PH-CO-CN} \qquad \text{I}$$

wobei PH einen Phenylrest bedeutet, der durch Chlormethyl oder Brommethyl substituiert ist und der gewünschtenfalls noch 1 bis 4 weitere, für die Umsetzung inerte Reste tragen kann,
dadurch gekennzeichnet, daß man ein Halogenmethylbenzoylchlorid der allgemeinen Formel II

$$\text{PH-CO-Cl} \qquad \text{II}$$

mit einem Alkalimetall- oder Übergangsmetallcyanid umsetzt.

2. Verfahren nach Anspruch 1, dadurch gekennzeichnet, daß man die Umsetzung in einem organischen Verdünnungsmittel vornimmt.

3. Verfahren nach Anspruch 1, dadurch gekennzeichnet, daß man die Umsetzung in Gegenwart eines Katalysators in einem organischen Verdunnungsmittel vornimmt.

4. Verfahren nach Anspruch 1, dadurch gekennzeichnet, daß man die Verbindungen II in einem organischen Verdünnungsmittel in Gegenwart eines Phasentransfer-Katalysators mit einer wässrigen Natrium- oder Kaliumcyanid-Lösung umsetzt.

5. Halogenmethyl-benzoylcyanide der Formel I'

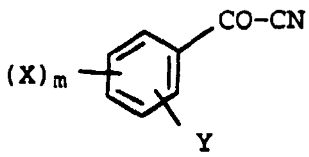

I'

wobei die Variablen die folgende Bedeutung haben

X Halogen, $C_1$-$C_4$-Alkyl, $C_1$-$C_4$-Alkoxy, Trifluormethyl, $C_1$-$C_5$-Alkyl-($C_1$-$C_5$-alkyl)hydroxyimino und $C_1$-$C_5$-Alkyl ($C_2$-$C_5$-alkenyl)hydroxyimino;

m 0 bis 4 und
Y Chlormethyl oder Brommethyl.

**6.** Halogenmethyl-benzoylcyanide der Formel I' nach Anspruch 5, wobei Y für 2-Chlormethyl und m für 0 steht.

## Claims

**1.** A process for preparing halomethylbenzoyl cyanides of the general formula I

Ph-CO-CN I

where Ph is a phenyl radical which is substituted by chloromethyl or bromomethyl and which, if desired, can additionally carry 1 to 4 further radicals which are inert to the reaction,

which comprises reacting a halomethylbenzoyl chloride of the general formula II

Ph-CO-Cl II

with an alkali metal cyanide or transition metal cyanide.

**2.** A process as claimed in claim 1, wherein the reaction is carried out in an organic diluent.

**3.** A process as claimed in claim 1, wherein the reaction is carried out in an organic diluent in the presence of a catalyst.

**4.** A process as claimed in claim 1, wherein the compounds II are reacted with an aqueous sodium cyanide or potassium cyanide solution in an organic diluent in the presence of a phasetransfer catalyst.

**5.** A halomethylbenzoyl cyanide of the formula I'

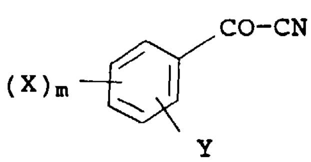

I'

where the variables have the following meanings

X is halogen, $C_1$-$C_4$-alky-, $C_1$-$C_4$-alkoxy, trifluoromethyl, $C_1$-$C_5$-alkyl-($C_1$-$C_5$-alkyl)hydroxyimino and $C_1$-$C_5$-alkyl($C_2$-$C_5$-alkenyl)hydroxyimino;

m is 0 to 4 and
y is chloromethyl or bromomethyl.

**6.** A halomethylbenzoyl cyanide of the formula I' as claimed in claim 5, where Y is 2-chloromethyl and m is 0.

## Revendications

**1.** Procédé pour la préparation de cyanures d'halogénométhyl-benzoyle de formule générale I

PH-CO-CN I

où PH représente un reste phényle, qui est substitué par un groupe chlorométhyle ou bromométhyle et qui, si on le souhaite, peut encore porter 1 à 4 autres restes inertes pour la réaction,
caractérisé par le fait qu'on fait réagir un chlorure d'halogénométhylbenzoyle de formule générale II

PH-CO-Cl II

avec un cyanure de métal alcalin ou de métal de transition.

**2.** Procédé selon la revendication 1, caractérisé par le fait qu'on effectue la conversion dans un agent de dilution organique.

**3.** Procédé selon la revendication 1, caractérisé par le fait qu'on effectue la conversion en présence d'un catalyseur dans un agent de dilution organique.

**4.** Procédé selon la revendication 1, caractérisé par le fait qu'on fait réagir les composés II dans un solvant organique, en présence d'un catalyseur de transfert de phase avec une solution aqueuse de cyanure de sodium ou de potassium.

**5.** Cyanures d'halogénométhyl-benzoyle de formule I'

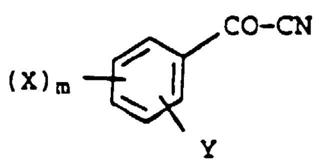

I'

où les variables ont les significations suivantes:

X est un groupe halogène, alkyle en $C_1$-$C_4$, alcoxy en $C_1$-$C_4$, trifluorométhyle, alkyl($C_1$-$C_5$)-(alkyl en $C_1$-$C_5$)-hydroxyimino et alkyl($C_1$-$C_5$)-(alcényl en $C_2$-$C_5$)-hydroxyimino;
m vaut 0 à 4 et
Y est un groupe chlorométhyle ou bromométhyle.

**6.** Cyanures d'halogénométhyl-benzoyle de formule I' selon la revendication 5, où Y désigne le groupe 2-chlorométhyle et m vaut 0.